# EUROPEAN PATENT APPLICATION

(11) **EP 4 502 150 A1**
(43) Date of publication of application: **05.02.2025**
(21) Application number: 23794824.5
(22) Date of filing: 15.03.2023
(51) Int. Cl.: C12N 5/10, C12N 15/85, A61K 35/17, A61P 35/00

(54) **ENGINEERED IMMUNE CELL WITH CD7 GENE KNOCK-OUT AND USE THEREOF**

(30) Priority: 28.04.2022 CN 202210460785
(71) Applicant: Nanjing Bioheng Biotech Co., Ltd, Nanjing, Jiangsu 210061 (CN)
(72) Inventor: ZHOU, Yali, Nanjing, Jiangsu 210061 (CN); CHEN, Gong, Nanjing, Jiangsu 210061 (CN); HUANG, Jie, Nanjing, Jiangsu 210061 (CN); ZHANG, Xuejian, Nanjing, Jiangsu 210061 (CN); REN, Jiangtao, Nanjing, Jiangsu 210061 (CN)
(74) Representative: Rimini, Rebecca
(86) International application number: PCT/CN2023/081600
(87) International publication number: WO 2023/207388

(57) **Abstract**

Disclosed herein are an engineered immune cell with CD7 gene knock-out and use thereof. According to the present invention, an sgRNA specifically targeting CD7 gene is designed and synthesized, which can accurately target CD7 gene to achieve gene knock-out with high knock-out efficiency. The provided sgRNA can be used for preparing a CD7-targeting engineered immune cell, and can be further used for preparing a CD7-targeting universal CAR-T cell

## Description

### Cross-reference to related applications

The present disclosure claims priority to the Chinese patent application with application number CN202210460785.5 and title "ENGINEERED IMMUNE CELL WITH CD7 GENE KNOCK-OUT AND USE THEREOF" filed with the Chinese Patent Office on April 28, 2022, the entire contents of which are incorporated herein by reference.

### Technical Field

The present disclosure belongs to the field of biomedicine. More specifically, the present disclosure relates to an engineered immune cell with CD7 gene knock-out and use thereof.

### Background Art

In recent years, cancer immunotherapy technology has developed rapidly, especially chimeric antigen receptor T cell (CAR-T)-related immunotherapy has achieved excellent clinical effects in the treatment of tumor diseases. In CAR-T cell immunotherapy, T cells are genetically modified in vitro to enable them to recognize tumor antigens, and after expanding to a certain number, they are infused back into the patient's body to kill cancer cells, thereby achieving the purpose of treating tumors.

CD7 is a cell surface glycoprotein with a molecular weight of about 40kDa and belongs to the immunoglobulin superfamily. CD7 is expressed in most T cells, NK cells, myeloid cells, T cell acute lymphoblastic leukemia/lymphoma, acute myeloblastic leukemia, and chronic granulocytic leukemia. It is reported that the CD7 molecule acts as a co-stimulatory signal during T cell activation by binding to its ligand K12/SECTM1. Furthermore, it is reported that mouse T progenitor cells with the CD7 molecule disruption still resulted in normal T cell development and homeostasis, indicating that CD7 does not appear to have critical impacts on T cell development and function, making it a very suitable therapeutic target for the treatment of T-cell acute lymphoblastic leukemia (TALL). Therefore, using CD7 as targets for cytotoxic molecules for the treatment of leukemia and lymphoma and applying it to CAR-T cell immunotherapy will have important application prospects in diseases related to CD7 expression.

However, since CD7 itself is also expressed in T cells, in order to avoid mutual killing between CAR-T cells, CD7 in T cells need to be knocked out first when preparing CD7-targeting CAR-T cells.

### Summary

The present disclosure aims to provide an sgRNA specifically targeting the CD7 gene and an engineered immune cell in which the CD7 gene is knocked out using the sgRNA, and its use in disease prevention and/or treatment and/or diagnosis and treatment of cancer, infection or autoimmune diseases.

In a first aspect, the present disclosure provides an sgRNA comprising a spacer sequence as set forth in any one of SEQ ID NOs: 1, 3, 4, 5, 8, and 9. Preferably, the spacer sequence is as set forth in any one of SEQ ID NOs: 1, 3, and 4.

In a second aspect, the present disclosure provides a nucleic acid encoding the above sgRNA and a vector comprising the nucleic acid.

In a third aspect, the present disclosure provides a method for knocking out the CD7 gene in vitro, comprising introducing a Cas nuclease and the above sgRNA into the cell.

In an embodiment, the Cas nuclease is Cas9, Cas12a or Cas13a, and is present in the form of a protein, an encoding nucleic acid thereof, or a vector thereof. Preferably, the Cas nuclease is Cas9.

In an embodiment, the sgRNA is present in the form of a RNA, an encoding nucleic acid or a vector thereof.

In an embodiment, the cell is an immune cell, and the immune cell is a T cell, a B cell, a macrophage, a dendritic cell, a monocyte, a NK cell, and/or a NKT cell and the like. Preferably, the immune cell is a T cell, a NK cell or a NKT cell. More preferably, the T cell is a CD4+CD8+ T cell, a CD4+ T cell, a CD8+ T cell, a memory T cell, a naive T cell, a γδ-T cell, and/or an αβ-T cell.

In an embodiment, the method further comprises introducing the nucleic acid encoding the chimeric antigen receptor or the T cell receptor or both into the immune cell.

In an embodiment, the chimeric antigen receptor comprises a ligand binding domain, a transmembrane domain, a co-stimulatory domain and a primary signaling domain.

In an embodiment, the ligand binding domain binds to the following targets: CD7, TSHR, CD19, CD123, CD22, BAFF-R, CD30, CD171, CS-1, CLL-1, CD33, EGFRvIII, GD2, GD3, BCMA, GPRC5D, Tn Ag, PSMA, ROR1, FLT3, FAP, TAG72, CD38, CD44v6, CEA, EPCAM, B7H3, KIT, IL-13Ra2, mesothelin, IL-1 IRa, PSCA, PRSS21, VEGFR2, LewisY, CD24, PDGFR-β, SSEA-4, CD20, Folate receptor α, ERBB2 (Her2/neu), MUC1, EGFR, NCAM, Claudin18.2, Prostase, PAP, ELF2M, Ephrin B2, IGF-I receptor, CAIX, LMP2, gploo, bcr-abl, tyrosinase, EphA2, Fucosyl GMI, sLe, GM3, TGS5, HMWMAA, o-acetyl-GD2, Folate receptor β, TEM1/CD248, TEM7R, CLDN6, GPRC5D, CXORF61, CD97, CD 179a, ALK, polysialic acid, PLAC1, GloboH, NY-BR-1, UPK2, HAVCR1, ADRB3, PANX3, GPR20, LY6K, OR51E2, TARP, WT1, NY-ESO-1, LAGE-la, MAGE-A1, legumin, HPV E6, E7, MAGE AI, ETV6-AML, sperm protein 17, XAGE1, Tie 2, MAD-CT-1, MAD-CT-2, Fos-related antigen 1, p53, p53 mutant, prostate-specific protein, survivin and telomerase, PCTA-1/Galectin 8, MelanA/MART1, Ras mutant, hTERT, sarcoma translocation breakpoint, ML-IAP, ERG (TMPRSS2 ETS fusion gene), NA17, PAX3, androgen receptor, Cyclin BI, MYCN, RhoC, TRP-2, CYP1B 1, BORIS, SART3, PAX5, OY-TES 1, LCK, AKAP-4, SSX2, RAGE-1, human telomerase reverse transcriptase, RU1, RU2, intestinal tract carboxylesterase, mut hsp70-2, CD79a, CD79b, CD72, LAIR1, FCAR, LILRA2, CD300LF, CLEC12A, BST2, EMR2, LY75, GPC3, FCRL5, IGLL1, PD1, PDL1, PDL2, TGFβ, APRIL, NKG2D or any combination thereof. Preferably, the target is selected from the group consisting of: CD7, CD19, CD20, CD22, BAFF-R, CD33, EGFRvIII, BCMA, GPRC5D, PSMA, ROR1, FAP, ERBB2 (Her2/neu), MUC1, EGFR, CAIX, WT1, NY-ESO-1, CD79a, CD79b, GPC3, Claudin18.2, NKG2D or any combination thereof. Those skilled in the art can determine the antigen to be targeted based on the condition to be treated, thereby designing the corresponding chimeric antigen receptor.

In an embodiment, the ligand binding domain comprises a CD7-targeting antibody or antigen binding fragment thereof. Preferably, the ligand binding domain of the present disclosure comprises a light chain variable region sequence having at least 90%, at least 95%, at least 97%, at least 99% or 100% sequence identity to the amino acid sequence as set forth in SEQ ID NO: 12, and a heavy chain variable region sequence having at least 90%, at least 95%, at least 97%, at least 99% or 100% sequence identity to the amino acid sequence as set forth in SEQ ID NO: 13.

In an embodiment, the transmembrane domain is a transmembrane domain of a protein selected from the group consisting of: a TCRα chain, a TCRβ chain, a TCRγ chain, a TCRδ chain, a CD3ζ subunit, a CD3ε subunit, a CD3γ subunit, a CD3δ subunit, CD45, CD4, CD5, CD8α, CD9, CD16, CD22, CD33, CD28, CD37, CD64, CD80, CD86, CD134, CD137, CD154 or any combination thereof. Preferably, the transmembrane domain is derived from a CD8α chain, which has at least 70%, preferably at least 80%, more preferably at least 90%, at least 95%, at least 97%, at least 99% or 100% sequence identity to the amino acid sequence as set forth in SEQ ID NO: 15, or the coding sequence of the CD8α transmembrane domain has at least 70%, preferably at least 80%, more preferably at least 90%, at least 95%, at least 97%, at least 99% or 100% sequence identity to the nucleotide sequence as set forth in SEQ ID NO: 16.

In an embodiment, the chimeric antigen receptor further comprises a hinge region located between the ligand binding domain and the transmembrane domain. Preferably, the hinge region comprises a hinge region of CD8α chain, FcγRIIIα receptor, IgG4 or IgG1, more preferably CD8α hinge region, which has at least 70%, preferably at least 80%, more preferably at least 90%, at least 95%, at least 97%, at least 99% or 100% sequence identity to the amino acid sequence as set forth in SEQ ID NO: 17, or the coding sequence of the CD8α hinge has at least 70%, preferably at least 80%, more preferably at least 90%, at least 95%, at least 97%, at least 99% or 100% sequence identity to the nucleotide sequence as set forth in SEQ ID NO: 18.

In an embodiment, the primary signaling domain is a signaling domains of a protein selected from the group consisting of: FcRγ, FcRβ, CD3γ, CD3δ, CD3ε, CD3ζ, CD22, CD79a, CD79b, CD66d, or any combination thereof. Preferably, the primary signaling domain comprises a CD3ζ primary signaling domain, which has at least at least 70%, preferably at least 80%, more preferably at least 90%, at least 95%, at least 97%, at least 99% or 100% sequence identity to the amino acid sequence as set forth in SEQ ID NO: 19 or 21, or the coding sequence thereof has at least 70%, preferably at least 80%, more preferably at least 90%, at least 95%, at least 97%, at least 99% or 100% sequence identity to the nucleotide sequence as set forth in SEQ ID NO: 20 or 22.

In an embodiment, the co-stimulatory domain includes but is not limited to a co-stimulatory signaling domain derived from the following proteins: TLR1, TLR2, TLR3, TLR4, TLR5, TLR6, TLR7, TLR8, TLR9, TLR10, CARD11, CD2, CD7, CD8, CD18 (LFA-1), CD27, CD28, CD30, CD40, CD54 (ICAM), CD83, CD134 (OX40), CD137 (4-1BB), CD150 (SLAMF1), CD152 (CTLA4), CD223 (LAG3), CD270 (HVEM), CD272 (BTLA), CD273 (PD-L2), CD274 (PD-L1), CD276 (B7-H3), CD278 (ICOS), CD357 (GITR), DAP10, LAT, NKG2C, SLP76, PD1, LIGHT, TRIM, ZAP70 or any combination thereof. Preferably, the co-stimulatory domain is selected from 4-1BB, CD28 or 4-1BB+CD28, more preferably 4-1BB co-stimulatory domain. The 4-1BB co-stimulatory domain has at least 70%, preferably at least 80%, more preferably at least 90%, at least 95%, at least 97%, at least 99% or 100% sequence identity to the amino acid sequence as set forth in SEQ ID NO: 23, or the coding sequence of the co-stimulatory domain has at least 70%, preferably at least 80%, more preferably at least 90%, at least 95%, at least 97%, at least 99% or 100% sequence identity to the nucleotide sequence as set forth in SEQ ID NO: 24.

In an embodiment, the chimeric antigen receptor further comprises a signal peptide, which has at least 70%, preferably at least 80%, more preferably at least 90%, at least 95%, at least 97% or at least 99% or 100% sequence identity to the amino acid sequence as set forth in SEQ ID NO: 25, or the coding sequence of the signal peptide has at least 70%, preferably at least 80%, more preferably at least 90%, at least 95%, at least 97% or at least 99% or 100% sequence identity to the nucleic acid molecule as set forth in SEQ ID NO: 26.

In an embodiment, the T cell receptor targets one or more of HBV, HPV E6, NYESO, mNY-ESO, WT1, MART-1, MAGE-A3, MAGE-A4, P53, Thyroglobulin, Tyrosinase.

In an embodiment, the chimeric antigen receptor or T cell receptor can be introduced into a host cell via a vector. Specifically, the vector is selected from the group consisting of plasmid, retrovirus, lentivirus, adenovirus, vaccinia virus, Rous sarcoma virus (RSV), polyoma virus and adeno-associated virus (AAV), phage, phagemid, cosmid or artificial chromosome. In some embodiments, the vector further comprises elements such as an origin of autonomous replication in host cells, a selectable marker, a restriction enzyme cleavage site, a promoter, a poly-A tail (polyA), a 3' UTR, a 5' UTR, an enhancer, a terminator, an insulator, an operon, a selectable marker, a reporter gene, a targeting sequence, and/or a protein purification tag. Specifically, the vector is a plasmid, a lentiviral vector, an AAV vector, an adenoviral vector or a retroviral vector.

In the fourth aspect, the present disclosure provides an engineered immune cell produced by the above method of knocking out the CD7 gene.

In an embodiment, the engineered immune cell further comprises suppressed or silenced expression of at least one gene selected from the group consisting of: GR, dCK, TCR/CD3 genes (e.g., TRAC, TRBC, CD3γ, CD3δ, CD3ε, CD3ζ), MHC related genes (HLA-A, HLA-B, HLA-C, B2M, HLA-DPA, HLA-DQ, HLA-DRA, TAP1, TAP2, LMP2, LMP7, RFX5, RFXAP, RFXANK, CIITA) and immune checkpoint genes such as PD1, LAG3, TIM3, CTLA4, PPP2CA, PPP2CB, PTPN6, PTPN22, PDCD1, HAVCR2, BTLA, CD160, TIGIT, CD96, CRTAM, TNFRSF10B, TNFRSF10A, CASP8, CASP10, CASP3, CASP6, CASP7, FADD, FAS, TGFBRII, TGFRBRI, SMAD2, SMAD3, SMAD4, SMAD10, SKI, SKIL, TGIF1, IL10RA, IL10RB, HMOX2, IL6R, IL6ST, EIF2AK4, CSK, PAG1, SIT, FOXP3, PRDM1, BATF, GUCY1A2, GUCY1A3, GUCY1B2 and GUCY1B3. Preferably, the engineered immune cell further comprises suppressed or silenced expression of at least one gene selected from the group consisting of: TRAC, TRBC, HLA-A, HLA-B, HLA-C, B2M, RFX5, RFXAP, RFXANK, CIITA, PD1, LAG3, TIM3, CTLA4, more preferably TRAC, TRBC, HLA-A, HLA-B, HLA-C, B2M, RFX5, RFXAP, RFXANK, CIITA. More preferably, CD7 and TRAC are knocked out in the engineered immune cell, and this inactivation makes the TCR-CD3 complex non-functional in the cell. This strategy is particularly useful for avoiding graft-versus-host disease (GvHD).

In a fifth aspect, the present disclosure further provides a composition comprising the above engineered immune cells, and the use of the immune cell or composition in the preparation of a medicament for treating/preventing/diagnosing cancer, infection or autoimmune disease.

In an embodiment, the composition further comprises one or more pharmaceutically acceptable excipients.

In an embodiment, the engineered immune cell or composition of the present disclosure may further be used in combination with one or more additional chemotherapeutic agents, biological agents, drugs or treatments. In this embodiment, the chemotherapeutic agent, biological agent, drug or treatment is selected from radiotherapy, surgery, antibody reagents, small molecules or any combination thereof.

The advantage of the present disclosure is that the screened sgRNA can efficiently knock out the CD7 gene, thereby avoiding mutual killing between CAR-T cells, and preparing universal CAR-T cells by simultaneously knocked out the TRAC gene.

The present disclosure will be described in detail with reference to the accompanying drawings and in combination with the examples. It should be noted that those skilled in the art should understand that the drawings and the embodiments of the present disclosure are only for the purpose of illustration, and shall not constitute any limitation to the present disclosure. In the case of no contradiction, the embodiments in the present application and the features in the embodiments can be combined with each other.

### Brief Description of Drawings

FIG. 1 shows the knock-out of the TRAC/CD7 gene in CD7 CAR-T cells with TRAC/CD7 double knock-out (CAR7-37KO).
FIG. 2 shows the expression level of CAR in CD7 single knock-out CD7 CAR-T cells (CAR7-7KO) and TRAC/CD7 double knock-out CD7 CAR-T cells (CAR7-37KO).
FIG. 3 shows the specific lysis rate of Jurkat target cells by CD7 single knock-out CD7 CAR-T cells (CAR7-7KO) and TRAC/CD7 double knock-out CD7 CAR-T cells (CAR7-37KO).
FIG. 4 shows the cytokine IFNγ secretion level of CD7 single knock-out CD7 CAR-T cells (CAR7-7KO) and TRAC/CD7 double knock-out CD7 CAR-T cells (CAR7-37KO).

### Detailed Description of Embodiments

### Detailed Description

Unless otherwise defined, all technical and scientific terms used herein have the same meaning as commonly understood by ordinary technicians in the field to which this application belongs. In order to make it easier to understand this application, certain terms are defined below.

### sgRNA

The CRISPR/Cas system is a natural immune system of prokaryotes. After being invaded by a virus, some bacteria can store a small segment of the viral gene in a storage space called CRISPR in their own DNA. When the virus invades again, the bacteria can recognize the virus based on the stored segment and cut off the viral DNA to render it ineffective. There are type I, type II and type III of CRISPR systems, among which CRISPR/Cas9 is the most studied as type II. In addition to Cas9 nuclease, Cas12a (also known as Cpf1) and Cas13a (also known as C2c2) are also commonly used Cas nucleases. CRISPR/Cas9-mediated gene editing technology can be used to generate transgenic models, regulate transcription, and regulate epigenetics, etc.

CRISPR/Cas9 system is mainly composed of Cas9 protein and single-stranded guide RNA (sgRNA), in which the Cas9 protein has the function of cutting DNA double-strand and the sgRNA plays a guiding role. The principle of this system is that CRISPR RNA (crRNA) bind to tracrRNA (trans-activating RNA) through base pairing to form tracrRNA/crRNA complex, which guides the nuclease Cas9 protein to cut double-stranded DNA at the sequence target site paired with the spacer sequence in the crRNA.

As used herein, the term "sgRNA", also known as "single guide RNA", refers to a guide RNA sequence used to target a specific gene so as to correct with CRISPR technology, which is usually composed by fusing crRNA and tracrRNA molecule that are partially complementary to form a complex, wherein the crRNA contains a sequence that is complementary to the target sequence so as to hybridize with the target sequence and guide the CRISPR complex (Cas9+crRNA+tracrRNA) to specifically bind to the target sequence. When the sgRNA binds to the Cas9 nuclease, it recognizes and binds to the DNA target site and causes a nick or cut (introduces a single-strand or double-strand break) in the DNA target site. It can recognize and cut the DNA target specific to the guide RNA. The part of the sgRNA responsible for complementary pairing with the target DNA is the spacer sequence contained therein. In an embodiment, the sgRNA comprises a spacer sequence as set forth in any one of SEQ ID NOs: 1, 3, 4, 5, 8, and 9.

The design of sgRNA spacer sequences is generally based on the following principles:
(1) The length should generally be around 20 nt;
(2) Base composition: the seed sequence (the 1st to 12th bases close to the PAM region) should avoid ending with more than 4 Ts, and the GC% content should be optimally 40% to 60%;
(3) The matching number between the seed sequence and the off-target site should be as low as possible;
(4) It is necessary to check whether there are SNPs in the genomic sequence of the target binding site;
(5) For the analysis of the off-target effect of the whole gene, the maximum allowable number of mismatched bases at the off-target site should be considered.

Therefore, the design of the sgRNA spacer needs to consider many factors, such as length, GC content, binding position of the target gene, binding rate with non-target sites, whether it contains SNPs, secondary structure, etc. At present, sgRNA can be designed through various online tools. However, since the Cas enzyme can cut any target sequence adjacent to the PAM site, for a specific target gene, the editing efficiency of a large number of sgRNAs designed by online tools is not the same, or even varies greatly. For example, the editing efficiency of the PAM site of 5'-NGG-3' is usually higher than that of 5'-NGA-3' or 5'-NAG-3'. Therefore, screening sgRNA with high specificity is crucial for improving the editing efficiency of CRISPR system.

### Nucleic acid, vector

As used herein, the term "nucleic acid" can be DNA or RNA.

As used herein, the term "vector" is a nucleic acid molecule used as a vehicle for transferring genetic material into a cell, in which the nucleic acid molecule can be replicated and/or expressed.

In an embodiment, the vectors of the present disclosure include, but are not limited to, linear nucleic acid (e.g., DNA or RNA), plasmid, virus (e.g., retrovirus, lentivirus, adenovirus, vaccinia virus, Rous sarcoma virus (RSV), polyoma virus, and adeno-associated virus (AAV), etc.), phage, phagemid, cosmid, and artificial chromosome (including BAC and YAC).

### Chimeric Antigen Receptor

As used herein, the term "chimeric antigen receptor" (CAR) comprises a ligand binding domain, a transmembrane domain, a co-stimulatory domain, and a primary signaling domain.

"Ligand binding domain" refers to any structure or functional variant thereof that can specifically bind to a ligand, including an antibody or antigen binding fragment thereof.

"Transmembrane domain" refers to a polypeptide structure that enables expression of a chimeric antigen receptor on the surface of an immune cell (e.g., a lymphocyte, a NK cell, or a NKT cell), and guides a cellular response of the immune cell against the target cell.

"Primary signaling domain" refers to a portion of a portion that transduces an effector function signal and guides a cell to perform a specified function, which is responsible for the intracellular primary signal transmission after the ligand binding domain binds to the antigen, thereby leading to the activation of immune cells and immune responses.

A "co-stimulatory domain" can be an intracellular functional signaling domain from a co-stimulatory molecule; A "co-stimulatory molecule" refers to a cognate binding partner that specifically binds to a co-stimulatory ligand on a T cell, thereby mediating a co-stimulatory response (e.g., proliferation) of the T cell.

As used herein, the transmembrane domain and the ligand binding domain are connected by a "hinge region" , which provides greater flexibility and accessibility for the ligand-binding domain. The hinge region may comprise up to 300 amino acids, preferably 10 to 100 amino acids and most preferably 25 to 50 amino acids.

In a preferred embodiment, the ligand binding domain comprises a CD7-targeting antibody or antigen binding fragment thereof. The CAR of the present disclosure, when expressed in immune cells, is capable of recognizing an antigen based on antigen binding specificity. After binding to a specific antigen, it affects tumor cells, causing them to fail to grow, causing them to die or being affected by other ways and resulting in a reduction or elimination of the patient's tumor burden. Preferably, the antigen binding domain is fused to a 4-1BB co-stimulatory domain and an intracellular domain combined with a CD3ζ signaling domain.

As used herein, the term "antibody" includes monoclonal antibodies (including whole antibodies), polyclonal antibodies, multivalent antibodies, multispecific antibodies (e.g., bispecific antibodies), and antibody fragments or synthetic polypeptides carrying one or more CDR sequences capable of exhibiting the desired biological activity. Typically, whole antibodies include two heavy chains and two light chains linked together by disulfide bonds, forming a "Y" shaped structure. Each of the heavy chain and light chain includes a variable region and a constant region, and each of the heavy and light chain variable regions includes three CDRs, which are separated by a more conservative framework region (FR). The variable region is responsible for the recognition and binding of the antigen, while the constant region can mediate the binding of the antibody to the host tissue or factor.

As used herein, the term "antigen-binding fragment" comprises only a portion of an intact antibody, and typically comprises the antigen-binding site of the intact antibody and thus retains the ability to bind antigen. Examples of antigen-binding fragments in the present disclosure include, but are not limited to: Fab, Fab', F(ab')2, Fd fragment, Fd', Fv fragment, scFv, disulfide-linked Fv (sdFv), antibody heavy chain variable region (VH) or light chain variable region (VL), linear antibody, "diabody" with two antigen binding sites, single domain antibody, nanobody, a natural ligand for the antigen or a functional fragment thereof.

The chimeric antigen receptor of the present disclosure may further comprise a signal peptide such that when it is expressed in a cell such as a T cell, the nascent protein is directed to the endoplasmic reticulum and subsequently to the cell surface. The core of the signal peptide may comprise a long hydrophobic amino acid segment, which has a tendency to form a single α-helix. Signal peptides that can be used in the present disclosure are well known to those skilled in the art, for example, signal peptides derived from CD8α, IgG1, GM-CSFRα, and the like.

### Engineered immune cells

As used herein, the term "immune cell" refers to any cell of the immune system that has one or more effector functions (e.g., cytotoxic cell killing activity, secretion of cytokines, induction of ADCC and/or CDC). For example, the immune cell can be a T cell, a macrophage, a dendritic cell, a monocyte, a NK cell, and/or a NKT cell. In an embodiment, the immune cell is derived from a stem cell, such as an adult stem cell, an embryonic stem cell, a cord blood stem cell, a progenitor cell, a bone marrow stem cell, an induced pluripotent stem cell, a totipotent stem cell, or a hematopoietic stem cell, and so on. Preferably, the immune cell is a T cell. The T cell can be any T cell, such as in vitro cultured T cell, for example, primary T cell, or T cell from in vitro cultured T cell line, e.g., Jurkat, SupT1, etc., or T cell obtained from a subject. Examples of subject include humans, dogs, cats, mice, rats, and transgenic species thereof. The T cell can be obtained from a variety of sources, including peripheral blood monocytes, bone marrow, lymph node tissue, umbilical blood, thymus tissue, tissue from sites of infection, ascites, pleural effusion, spleen tissue, and tumors. The T cell also can be concentrated or purified. The T cell can be at any stage of development including, but not limited to, a CD4⁺/CD8⁺ T cell, a CD4⁺ helper T cell (e.g., Th1 cell and Th2 cell), a CD8⁺ T cell (e.g., cytotoxic T cell), a tumor infiltrating cell, a memory T cell, a naive T cell, a γδ-T cell, an αβ-T cell. In a preferred embodiment, the immune cell is a human T cell. The T cell can be isolated from the blood of a subject using a variety of techniques known to those of skill in the art, such as Ficoll.

In an embodiment, in order to reduce the risk of graft-versus-host disease, the engineered immune cell further comprises suppressed or silenced expression of at least one gene selected from the group consisting of: CD52, GR, dCK, TCR/CD3 genes (e.g., TRAC, TRBC, CD3γ, CD3δ, CD3ε, CD3ζ), MHC related genes (HLA-A, HLA-B, HLA-C, B2M, HLA-DPA, HLA-DQ, HLA-DRA, TAP1, TAP2, LMP2, LMP7, RFX5, RFXAP, RFXANK, CIITA) and immune checkpoint genes such as PD1, LAG3, TIM3, CTLA4, PPP2CA, PPP2CB, PTPN6, PTPN22, PDCD1, HAVCR2, BTLA, CD160, TIGIT, CD96, CRTAM, TNFRSF10B, TNFRSF10A, CASP8, CASP10, CASP3, CASP6, CASP7, FADD, FAS, TGFBRII, TGFRBRI, SMAD2, SMAD3, SMAD4, SMAD10, SKI, SKIL, TGIF1, IL10RA, IL10RB, HMOX2, IL6R, IL6ST, EIF2AK4, CSK, PAG1, SIT, FOXP3, PRDM1, BATF, GUCY1A2, GUCY1A3, GUCY1B2 and GUCY1B3. Preferably, the engineered immune cell further comprises suppressed or silenced expression of at least one gene selected from the group consisting of: TRAC, TRBC, HLA-A, HLA-B, HLA-C, B2M, RFX5, RFXAP, RFXANK, CIITA, PD1, LAG3, TIM3, CTLA4, more preferably TRAC, TRBC, HLA-A, HLA-B, HLA-C, B2M, RFX5, RFXAP, RFXANK, CIITA.

### Composition

The present disclosure further provides a composition comprising the engineered immune cell body described in the present disclosure. In an embodiment, the composition further comprises one or more pharmaceutically acceptable excipients.

As used herein, the term "pharmaceutically acceptable excipient" refers to carriers and/or excipients that are pharmacologically and/or physiologically compatible with the subject and the active ingredient (i.e., capable of inducing the desired therapeutic effect without causing any undesirable local or systemic effects), including but not limited to , filler, binder, disintegrant, coating agent, adsorbent, anti-adherent, glidant, antioxidant, flavoring agent, colorant, sweetener, solvent, co-solvent, buffer agent, chelating agent, surfactant, diluent, wetting agent, preservative, emulsifier, cladding agent, isotonic agent, absorption delaying agent, stabilizer, and tension regulator. There are many ways of administering the composition in the art, including but not limited to oral, injection, aerosol, parenteral and topical administration.

The composition of the present disclosure can further be administered in combination with one or more other agents suitable for treating and/or preventing the disease to be treated. Preferred examples of agent suitable for the combination include known anti-cancer medicines such as cisplatin, maytansine derivatives, rachelmycin, calicheamicin, docetaxel, etoposide, gemcitabine, ifosfamide, irinotecan, melphalan, mitoxantrone, sorfimer sodiumphotofrin II, temozolomide, topotecan, trimetreate glucuronate, auristatin E, vincristine and doxorubicin; peptide cytotoxins, such as ricin, diphtheria toxin, pseudomonas exotoxin A, DNase and RNase; radionuclides such as iodine 131, rhenium 186, indium 111, iridium 90, bismuth 210, bismuth 213, actinides 225 and astatine 213; prodrugs such as antibody-directed enzyme prodrugs; immunostimulatory agents such as platelet factor 4, and melanoma growth stimulating protein; antibodies or fragments thereof, such as anti-CD3 antibodies or fragments thereof, complement activators, heterologous protein domains, homologous protein domains, viral/bacterial protein domains and viral/bacterial peptides. In addition, the pharmaceutical composition of the present disclosure also can be used in combination with one or more other treatment methods, such as chemotherapy and radiotherapy.

### Therapeutic/preventive/diagnostic use

The engineered immune cells and compositions of the present disclosure can be used as drugs for the treatment/prevention/diagnosis of cancer, infection or autoimmune diseases.

In an embodiment, cancers that can be treated/prevented/diagnosed with the engineered immune cells or compositions of the present disclosure include non-solid tumors (such as hematological tumors, e.g., leukemias and lymphomas) and solid tumors. Hematological tumors are cancers of the blood or bone marrow, including but not limited to acute leukemias (such as acute lymphoblastic leukemia, acute myeloid leukemia, acute myelogenous leukemia and myeloblastic, promyelocytic, granulocytic-monocytic, monocytic and erythroleukemia), chronic leukemias (such as chronic myeloid (granulocytic) leukemia, chronic myelogenous leukemia and chronic lymphocytic leukemia), polycythemia vera, lymphomas, Hodgkin lymphoma, non-Hodgkin lymphoma (indolent and high-grade forms), multiple myeloma, Waldenstrom's macroglobulinemia, myelodysplastic syndrome, hairy cell leukemia, Burkitt lymphoma, diffuse large cell lymphoma, mantle cell lymphoma, T-lymphoblastic leukemia/lymphoma (T-ALL/LBL), early pro-T lymphoblastic leukemia (ETP-ALL), extranodal NK/T-cell lymphoma, small lymphocytic lymphoma (SLL), and myelodysplasia. Solid tumors are abnormal masses of tissue that usually do not comprise cysts or fluid areas, and can be benign or malignant. Different types of solid tumors are named according to the type of cells from which they are derived (such as sarcomas, carcinomas and lymphomas). Examples of solid tumors include, but are not limited to, fibrosarcoma, myxosarcoma, liposarcoma, mesothelioma, pancreatic cancer, ovarian cancer, peritoneal, omental and mesenteric cancer, pharyngeal cancer, prostate cancer, rectal cancer, renal cancer, skin cancer, small intestine cancer, melanoma, kidney cancer, laryngeal cancer, soft tissue cancer, stomach cancer, testicular cancer, colon cancer, esophageal cancer, cervical cancer, alveolar rhabdomyosarcoma, bladder cancer, bone cancer, brain cancer, breast cancer, anal cancer, eye cancer, intrahepatic cholangiocarcinoma, joint cancer, neck cancer, gallbladder cancer, pleural cancer, nasal cancer, middle ear cancer, oral cancer, vulvar cancer, thyroid cancer and ureteral cancer. Preferably, cancers that can be treated/prevented/diagnosed with the engineered immune cells or compositions of the present disclosure include acute lymphoblastic leukemia (ALL), acute myeloid leukemia (AML), T-lymphoblastic lymphoma (T-LBL), early pre-T lymphoblastic leukemia (ETP-ALL) and extranodal NK/T cell lymphoma.

### Example 1. Design of sgRNAs and knock-out of CD7 gene

Suitable PAM sites on the CD7 gene were selected to design the spacer sequence of sgRNA. Spacer sequences target the exons of the CD7 gene, which is the unique target sequence on the CD7 gene. The crRNA containing the spacer sequence was fused with the tracrRNA to obtain a sequence of about 100 bp, which was artificially synthesized to obtain the sgRNA product.

T cells were stimulated with DynaBeads CD3/CD28 CTSTM (Gibco, Cat. No. 40203D) and cultured at 37°C and 5% CO2 for 3 days. Then, 5 µg of Cas9 protein and 5 µg of sgRNA were electrotransfected into the activated T cells using a BTX Agile Pulse Max electroporator (Harvard Apparatus BTX) at 400V and 0.7ms to obtain T cells with CD7 knock-out. After electrotransfection, the T cells were immediately placed in 1 ml of preheated culture medium and cultured at 37°C and 5% CO2 in the presence of IL-2 (300 IU/ml). Five days later, the expression of CD7 was detected by flow cytometry using PE anti-human CD7 (Biolegend, Cat. No. 343106) to obtain the knock-out efficiency of the CD7 gene. The spacer sequences of different sgRNAs and their knock-out efficiencies are shown in Table 1.

**Table 1. Spacer sequences of different sgRNAs**

| No. | SEQ ID NO | Spacer sequence | Knock-out efficiency |
|---|---|---|---|
| 7-1 | 1 | GUCACGGAGGUCAAUGUCUA | 91.9% |
| 7-2 | 2 | GUAGACAUUGACCUCCGUGA | 77.4% |
| 7-3 | 3 | GAGGUCAAUGUCUACGGCUC | 94.0% |
| 7-4 | 4 | GGUUGACGGAGGCUCCCACG | 94.0% |
| 7-5 | 5 | GGUACGGACAGACGGUUCCG | 82.9% |
| 7-6 | 6 | GGGACCCUGAGAAGUCGAUG | 76.7% |
| 7-7 | 7 | GGAGCAGGUGAUGUUGACGG | 75.7% |
| 7-8 | 8 | GUGGCGGGAUAAGAAUUCGG | 85.6% |
| 7-9 | 9 | GUAGACAUUGACCUCCGUGA | 85.5% |
| 7-10 | 10 | GGAGCAGGUGAUGUUGACGG | 75.1% |

It can be seen that different sgRNAs have different knock-out efficiencies for the CD7 gene, among which the knock-out efficiencies of SEQ ID NO: 1, 3, 4, 5, 8, and 9 are all higher than 80%, and the knock-out efficiencies of SEQ ID NO: 1, 3, and 4 are even higher than 90%. Based on the knock-out efficiency, No. 7-4 was selected for subsequent experiments.

### Example 2. Preparation of CAR-T cells with CD7 and TRAC/CD7 knock-out

### 1. Preparation of CD7 CAR-T cells

The sequences coding the following proteins were synthesized and cloned sequentially into the pGEM-T Easy vector (Promega, Cat. No. A1360) in sequence: CD8α signal peptide (SEQ ID NO: 25), anti-CD7 scFv (SEQ ID NO: 14), CD8α hinge region (SEQ ID NO: 17), CD8α transmembrane region (SEQ ID NO: 15), 4-1BB co-stimulatory domain (SEQ ID NO: 23), CD3ζ primary signaling domain (SEQ ID NO: 19), and the correct insertion of the target sequence was confirmed by sequencing.

3 ml Opti-MEM (Gibco, Cat. No. 31985-070) was added to a sterile tube to dilute the above plasmid, and then packaging vector psPAX2 (Addgene, Cat. No. 12260) and envelope vector pMD2.G (Addgene, Cat. No. 12259) were added according to the ratio of plasmid: viral packaging vector: viral envelope vector = 4:2:1. Then, 120 µl X-treme GENE HP DNA transfection reagent (Roche, Cat. No. 06366236001) was added, mixed immediately, and incubated at room temperature for 15 min, and then the plasmid/vector/transfection reagent mixture was added dropwise to the culture flask containing 293T cells. Viruses were collected at 24 hours and 48 hours, pooled, and ultracentrifuged (25000g, 4°C, 2.5 hours) to obtain concentrated lentivirus.

T cells were activated with DynaBeads CD3/CD28 CTSTM (Gibco, Cat. No. 40203D), and were further cultured for 1 day at 37°C and 5% CO₂. Then, the concentrated lentivirus was added, and after 3 days of continuous culture, CD7-targeting CAR-T cells were obtained.

Using a BTX Agile Pulse Max electroporator (Harvard Apparatus BTX), 10 µg of Cas9 protein, 5 µg of CD7 sgRNA (spacer sequence as as set forth in SEQ ID NO: 4) or a combination thereof with 5 µg of TRAC sgRNA (spacer sequence as as set forth in SEQ ID NO: 11) were electrotransfected into activated CD7 CAR T cells at 400V and 0.7ms to obtain single knock-out (CAR7-7KO) and TRAC/CD7 double knock-out (CAR7-37KO) CD7 CAR-T cells. Wild-type T cells without CAR transfection were used as negative controls (NT).

### 2. Editing efficiency and CAR level detection

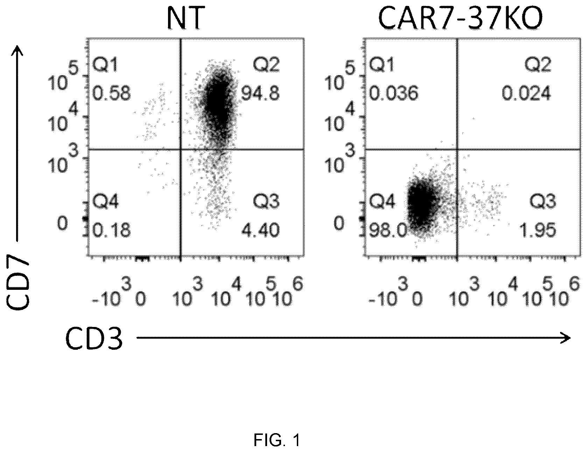

After culturing CAR7-7KO and CAR7-37KO cells at 37°C and 5% CO2 for 11 days, the gene editing efficiency of TRAC and CD7 was detected using APC-anti human CD3 (BD Pharmingen, Catalog No. 555751) and PE anti-human CD7 (Biolegend, Catalog No. 343106) antibodies by flow cytometry, and the results are shown in Figure 1.

It can be seen that the target gene was effectively knocked out in CAR7-37KO cells, indicating that the selected sgRNA of the present disclosure can effectively knock out the TRAC and CD7 genes.

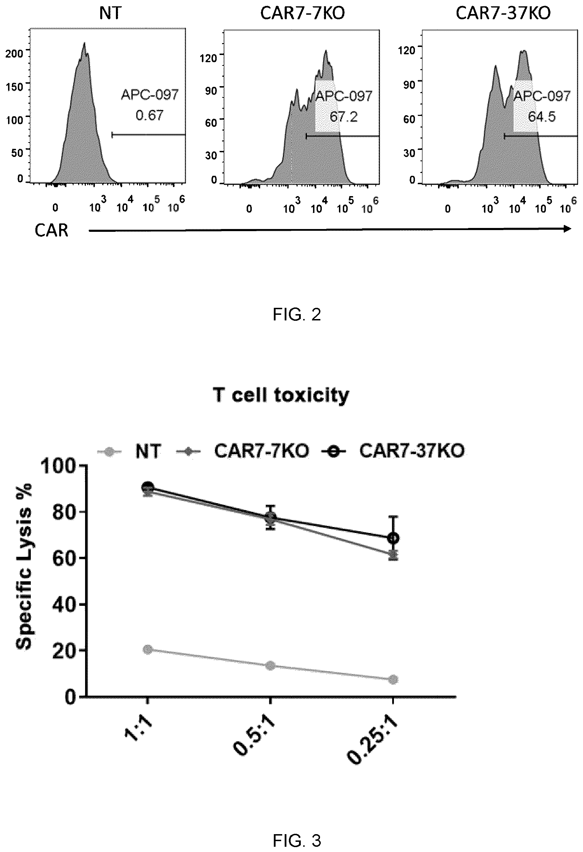

In addition, after culturing CAR7-7KO and CAR7-37KO cells at 37°C and 5% CO2 for 11 days, the expression level of CAR on CAR T cells was detected using Biotin-SP (long spacer) AffiniPure Goat Anti-Mouse IgG, F(ab')₂ Fragment Specific(min X Hu, Bov, Hrs Sr Prot) (jackson immunoresearch, Cat. No. 115-065-072) as the primary antibody, APC Streptavidin (BD Pharmingen, Cat. No. 554067) or PE Streptavidin (BD Pharmingen, Cat. No. 554061) as the secondary antibody by flow cytometry, and the results are shown in Figure 2.

It can be seen that the expression level of CAR in CAR7-7KO is comparable to that in CAR7-37KO.

### Example 3. Killing effect and cytokine secretion level of CAR-T cells

### 1. Killing effect on target cells

In order to detect the killing ability of CAR-T cells to target cells, Jurkat target cells carrying fluorescein gene were first plated into a 96-well plate at 1×10⁴/well, and then three groups of cells were plated into the 96-well plate with different effector-target ratios (i.e., the ratio of effector T cells to target cells) for co-culture, and the fluorescence value was measured with a microplate reader after 8 hours. According to the calculation formula: (average fluorescence value of target cells - average fluorescence value of samples)/average fluorescence value of target cells × 100%, the killing efficiency was calculated, and the results are shown in Figure 3.

It can be seen that compared with NT, cells in the CAR7-7KO and CAR7-37KO groups can effectively kill target cells, and there is no significant difference in the killing effect between the two groups.

### 2. Secretion level of Cytokine

When T cells kill target cells, the number of target cells decreases and cytokines such as IL2 and IFN-γ are released at the same time. According to the following steps, enzyme-linked immunosorbent assay (ELISA) was used to measure the release level of cytokine IFNγ when CAR T cells killed target cells.

### (1) Collection of cell co-culture supernatant

Jurkat target cells were plated in 96-well plates at 1×10⁵/well, and then the three groups of cells were co-cultured with target cells at a ratio of 1:1, and cell co-culture supernatant was collected after 8 hours.

### (2) Detection of the secretion of IFN-γ in the supernatant by ELISA

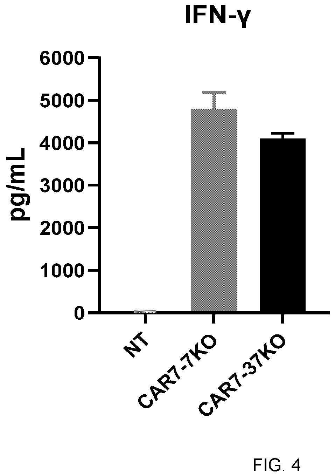

The secretion of cytokine IFNγ in the supernatant was measured by ELISA. A 96-well plate was coated with Purified anti-human IFN-γ Antibody (Biolegend, Cat. No. 506502) as capture antibody and incubated overnight at 4°C, and then the antibody solution was removed. 250 µL of PBST (1XPBS containing 0.1% Tween) solution containing 2% BSA (sigma, Cat. No. V900933-1kg) was added, and incubated at 37°C for 2 hours. Then, 50 µL of cell co-culture supernatant or standard was added to each well and incubated at 37°C for 1 hour. After washing the plate with 250 µL PBST (1XPBS containing 0.1% Tween), 50 µL of detection antibody Anti-Interferon gamma antibody [MD-1] (Biotin) (abcam, Cat. No. ab25017) was added to each well and incubated at 37°C for 1 hour. Then HRP Streptavidin (Biolegend, Cat. No. 405210) was added, incubated at 37°C for 30 minutes, and then the supernatant was discarded, washed with 250 µL PBST (1XPBS containing 0.1% Tween). 50 µL of TMB substrate solution was added to each well. Reactions were allowed to occur at room temperature in the dark for 30 minutes, after which 50 µL of 1 mol/L H₂SO₄ was added to each well to stop the reaction. Within 30 minutes after the reaction stopped, a microplate reader was used to detect the absorbance at 450 nm, and the content of cytokines was calculated according to the standard curve (plotted according to the reading value and concentration of the standard), the results are shown in Figure 4.

It can be seen that compared with the NT group, both CAR7-7KO cells and CAR7-37KO cells have a large amount of cytokine secretion.

It should be noted that the above-mentioned are merely for preferred examples of the present disclosure and not used to limit the present disclosure. For one skilled in the art, various modifications and changes may be made to the present disclosure. Those skilled in the art should understand that any amendments, equivalent replacements, improvements, and so on, made within the spirit and principle of the present disclosure, should be covered within the scope of protection of the present disclosure.

## Claims

1. An sgRNA comprising a spacer sequence as set forth in any one of SEQ ID NOs 1, 3, 4, 5, 8, and 9.

2. A nucleic acid encoding the sgRNA of claim 1.

3. A vector comprising the nucleic acid of claim 2.

4. A method for knocking out the CD7 gene *in vitro,* comprising introducing a Cas nuclease and a sgRNA into a cell, **characterized in that** the sgRNA is in the form of the RNA of claim 1, the nucleic acid of claim 2, or the vector of claim 3.

5. The method of claim 4, **characterized in that** the Cas nuclease is Cas9, Cas12a, or Cas13a.

6. The method of claim 4, **characterized in that** the cell is an immune cell, and the immune cell is a T cell, a B cell, a macrophage, a dendritic cell, a monocyte, a NK cell, or a NKT cell.

7. The method of claim 6, **characterized in that** the cell is a CD4⁺CD8⁺ T cell, a CD4⁺ T cell, a CD8⁺ T cell, a memory T cell, a naive T cell, a γδ-T cell, or an αβ-T cell.

8. The method of claim 6, **characterized in that** the immune cell is introduced with a nucleic acid encoding a chimeric antigen receptor and/or a T cell receptor.

9. The method of claim 8, **characterized in that** the chimeric antigen receptor comprises a ligand binding domain, a transmembrane domain, a co-stimulatory domain and a primary signaling domain, wherein the ligand binding domain targets one or more of CD7, CD19, CD20, CD22, BAFF-R, CD33, EGFRvIII, BCMA, GPRC5D, PSMA, ROR1, FAP, ERBB2, MUC1, EGFR, CAIX, WT1, NY-ESO-1, CD79a, CD79b, GPC3, Claudin18.2, and NKG2D.

10. The method of claim 9, **characterized in that** the ligand binding domain comprises a CD7-targeting antibody or antigen binding fragment thereof.

11. The method of claim 10, **characterized in that** the CD7-targeting antibody or antigen binding fragment thereof comprises a light chain variable region sequence having at least 90%, at least 95%, at least 97%, at least 99% or 100% sequence identity to the amino acid sequence as set forth in SEQ ID NO: 12 and a heavy chain variable region sequence having at least 90%, at least 95%, at least 97%, at least 99% or 100% sequence identity to the amino acid sequence as set forth in SEQ ID NO: 13.

12. The method of claim 9, **characterized in that** the transmembrane domain is a transmembrane domain of a protein selected from the group consisting of: a TCRα chain, a TCRβ chain, a TCRγ chain, a TCRδ chain, a CD3ζ subunit, a CD3ε subunit, a CD3γ subunit, a CD3δ subunit, CD45, CD4, CD5, CD8α, CD9, CD16, CD22, CD33, CD28, CD37, CD64, CD80, CD86, CD134, CD137, CD154 or any combination thereof.

13. The method of claim 9, **characterized in that** the co-stimulatory domain is a co-stimulatory signaling domain of a protein selected from the group consisting of: TLR1, TLR2, TLR3, TLR4, TLR5, TLR6, TLR7, TLR8, TLR9, TLR10, CARD11, CD2, CD7, CD8, CD18, CD27, CD28, CD30, CD40, CD54, CD83, CD134, CD137, CD150, CD152, CD223, CD270, CD272, CD273, CD274, CD276, CD278, CD357, DAP10, LAT, NKG2C, SLP76, PD1, LIGHT, TRIM, ZAP70 or any combination thereof.

14. The method of claim 9, **characterized in that** the primary signaling domain is a signaling domains of a protein selected from the group consisting of: FcRγ, FcRβ, CD3γ, CD3δ, CD3ε, CD3ζ, CD22, CD79a, CD79b, CD66d or any combination thereof.

15. An engineered immune cell obtained by the method of any one of claims 6-14, **characterized in that** the CD7 gene is knocked out.

16. The engineered immune cell of claim 15, **characterized in that** the engineered immune cell further comprises suppressed or silenced expression of at least one gene selected from the group consisting of: TRAC, TRBC, HLA-A, HLA-B, HLA-C, B2M, RFX5, RFXAP, RFXANK, CIITA, PD1, LAG3, TIM3, CTLA4 or any combination thereof.

17. The engineered immune cell of claim 16, **characterized in that** CD7 and TRAC in the engineered immune cell are knocked out.

18. A composition comprising the engineered immune cell of any one of claims 15-17.

19. Use of the engineered immune cell of any one of claims 15-17 or the composition of claim 18 in the preparation of a medicament for treating/preventing/diagnosing cancer, infection or autoimmune disease.
